# EUROPEAN PATENT APPLICATION

(11) **EP 1 923 806 A1**
(43) Date of publication of application: **21.05.2008**
(21) Application number: 06123995.0
(22) Date of filing: 14.11.2006
(51) Int. Cl.: G06F 19/00, G01N 33/50

(54) **Fast metabolomic analysis and system therefor**

(71) Applicant: Metanomics GmbH, 10589 Berlin (DE)
(72) Inventor: Fuchs, Regine, Dr., 10115 Berlin (DE); Looser, Ralf, Dr, 13158 Berlin (DE); Herold, Michael Manfred, Dr, 10551 Berlin (DE); Krotzky, Arno, Dr., 14542 Werder (DE); Dostler, Martin, 16761 Henningsdorf (DE); Prokoudine, Alexandre, Dr., 13189 Berlin (DE); Klein, Mathieu, Dr, 12159 Berlin (DE)
(74) Representative: Dick, Alexander

(57) **Abstract**

The present invention relates to a system for determining a physiological status, such as the general health status or a disease, comprising means for determining metabolic data, means for converting the metabolic data in an input format, at least one database comprising reference metabolic data into a format which allows comparison to the input format, means for comparing the metabolic data in the input format to the reference metabolic data comprised by the at least one database and means for converting the results of a comparison carried out by the aforementioned means into an output format. Moreover, the present invention relates to a method for determining a physiological status comprising determining metabolic data, converting the metabolic data in an input format, comparing the metabolic data with reference metabolic data which are indicative for the physiological status which are stored in at least one database and converting the results of the aforementioned comparison into an output format.

## Description

The present invention relates to a system for determining a physiological status, such as the general health status or a disease, comprising means for determining metabolic data, means for converting the metabolic data in an input format, at least one database comprising reference metabolic data into a format which allows comparison to the input format, means for comparing the metabolic data in the input format to the reference metabolic data comprised by the at least one database and means for converting the results of a comparison carried out by the aforementioned means into an output format. Moreover, the present invention relates to a method for determining a physiological status comprising determining metabolic data, converting the metabolic data in an input format, comparing the metabolic data with reference metabolic data which are indicative for the physiological status which are stored in at least one database and converting the results of the aforementioned comparison into an output format.

The currently available diagnostic systems and methods can be subdivided in two groups.

The first group encompasses diagnostic systems and methods which can be carried out in close proximity to the subject to be diagnosed. Such diagnostic systems and methods are, for example, portable systems for near-patient diagnosis or point-of-care diagnostics. For such a diagnostic approach, usually systems are used which operate in close proximity to the patient and which, once provided with a sample or a comparable input, deliver immediately an output to be used for the diagnosis. It is to be understood that such near patient systems usually aim to determine, if at all, merely a limited number of different analytes. Most of said systems merely determine a single analyte, such as glucose in the case of the commercially available diabetes test stripes. Moreover, the evaluation capacity of such systems is usually limited, too. In most cases, not even a precise value or amount may be provided. Rather, the systems may merely indicate the presence or absence of an analyte. Thus, a drawback of said portable systems is that more complex patterns of analytes and relative changes cannot be efficiently and reliably evaluated.

The second group of systems and methods are characterized in that they allow a comprehensive determination and evaluation of complex analyte pattern. However, such systems are rather cost-intensive and usually do not allow for a quick determination of specific analytes prior to the evaluation of the entire analyte pattern.

In particular, the aforementioned drawbacks occur in metabolic analysis. The analysis of the metabolome (often called "metabolomics") of an organism, i.e. the entirety of metabolites at a certain time and under certain conditions, usually requires the determination of a complex pattern of different compounds, i.e. of the metabolites. Nevertheless, metabolomics turned out recently to be a powerful tool for diagnostic approaches towards the physiological status of an individual, e.g., for diagnosing diseases or disorders.

Systems and methods which comply with the aforementioned needs are not yet available, but are, nevertheless, highly desirable.

The technical problem underlying the present invention, therefore, must be seen in the provision of means and methods which comply with the aforementioned needs. The technical problem is solved by the embodiments characterized herein below and in the accompanying claims.

Thus, the present invention relates to a system for determining a physiological status of a subject comprising operatively linked to each other:
(a) means for determining metabolic data from a sample of a subject whose physiological status is to be determined;
(b) means for converting the metabolic data into an input format;
(c) at least one database comprising reference metabolic data in a format which allows comparison to the input format;
(d) means for comparing the metabolic data in the input format to the reference metabolic data comprised by the at least one database; and
(e) means for converting the results of a comparison carried out by the means of (d) into an output format.

The term "system" as used in accordance with the present invention relates to a plurality of components (i.e. means), such as devices or databases, which are operatively linked to each other. The individual components of the system of the present invention are recited above and will be described in more detail below.

However, it is not necessary that the components of the system are in physical proximity to each other. For example, devices and databases may be operatively linked by wire or even wireless in physical distance, e.g. at separate locations even in separate countries of the world. Moreover, the databases and devices may be linked to each other via the internet. The terms "internet" and "world wide web (www)" are used herein interchangeably.

Further, it is to be understood that the components of the system do not have to be permanently linked to each other. For example, a device such as an apparatus for the determination of metabolic data may contact - and subsequently undergo operative linkage - to a database only after the metabolic data have been determined. During evaluation of said data using the database, the linkage may than be disrupted. After evaluation, the database may be in operative linkage with a suitable output device such as a computer terminal.

The data which are transferred between the components of the system are, preferably, kept confidential by suitable encryption techniques well known in the art.

Moreover, it is also preferably envisaged that the results provided in an output format are only available with a specific access key. The use of the system shall, preferably, depend on the payment of a user fee. Suitable means for allowing access to the system only upon payment of a user fee are well known in the art.

The term "physiological status" as used herein refers to the health status of a subject. Accordingly, the physiological status of the subject will indicate whether the subject is healthy or whether it suffers from a disease or disorder or any other medical condition, or whether is has a predisposition for a certain disease. Moreover, the physiological status may indicate the training status of a subject or its nutrient or supplement (e.g., vitamin) status. Preferably, the physiological status to be determined by the system of the present invention is a disease status, more preferably a disease status indicative for a disease select from the group consisting of: diabetes, cancer, metabolic disorders, diseases of the endocrine organs, neurological diseases and disorders, renal diseases and disorders, digestive system disease and disorders, bacterial or viral infections and cardiovascular diseases. The term "disease" as used herein below shall also encompass the aforementioned disorders or medical conditions.

The term "determining a physiological status" as used herein refers to assessing whether a subject exhibits the physiological status, e.g., whether the subject is healthy or suffers from a disease or disorder or a predisposition therefore. Preferably, determining as used herein encompasses diagnosing a disease status. In case of plants "determining a physiological status" as used herein preferably refers to assessing whether plant growth, development and seed or fruit production is compromised by any biotic or abiotic factors. Biotic factors specifically include various diseases or pathogens, like virus, fungal, bacterial nematode, aphides worms or insects. Abiotic factors comprise abiotic stresses like drought, cold, water stress, growth density, limited nutrients like, eg. Nitrogen, potassium, phosphor.

Diagnosing as used herein relates to assessing the probability according to which a subject is suffering or will suffer from a disease. As will be understood by those skilled in the art, such an assessment is usually not intended to be correct for 100% of the subjects to be diagnosed. The term, however, requires that a statistically significant portion of subjects can be diagnosed to suffer from the diseases. Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

Diagnosing according to the present invention includes monitoring, confirmation, subclassification and prediction of the relevant disease, symptoms or risks therefor. Monitoring relates to keeping track of an already diagnosed disease. It is to be understood that the success of a therapy can be also monitored by the method of the present invention. Confirmation relates to the strengthening or substantiating a diagnosis already performed using other indicators or markers. Subclassification relates to further defining a diagnosis according to different subclasses of the diagnosed disease, e.g. defining according to mild and severe forms of the disease. Prediction relates to prognosing a disease before other symptoms or markers have become evident or have become significantly altered.

The term "means for determining metabolic data" refers to devices which can be used to determine a metabolite or preferably a plurality of metabolites in a sample qualitatively or quantitatively. Qualitative determination as used herein means to determine the presence or absence of the metabolite. Quantitative determination relates to determining the relative or absolute amount of a metabolite present in a sample.

The metabolites referred to in accordance with the present invention are small molecule compounds, such as substrates for enzymes of metabolic pathways, intermediates of such pathways or the products obtained by a metabolic pathway. Metabolic pathways are well known in the art and may vary between species. Preferably, said pathways include at least citric acid cycle, respiratory chain, photosynthesis, photorespiration, glycolysis, gluconeogenesis, hexose monophosphate pathway, oxidative pentose phosphate pathway, production and β-oxidation of fatty acids, urea cycle, amino acid biosynthesis pathways, protein degradation pathways such as proteasomal degradation, amino acid degrading pathways, biosynthesis or degradation of: lipids, polyketides (including e.g. flavonoids and isoflavonoids), isoprenoids (including eg. terpenes, sterols, steroids, carotenoids, xanthophylls), carbohydrates, phenylpropanoids and derivatives, alkaloids, benzenoids, indoles, indole-sulfur compounds, porphyrines, anthocyans, hormones, vitamins, cofactors such as prosthetic groups or electron carriers, lignin, glucosinolates, purines, pyrimidines, nucleosides, nucleotides and related molecules such as tRNAs, microRNAs (miRNA) or mRNAs. Wir sollten überlegen, ob wir die RNAs ausklammern, da es möglicherweise für den Bereich Arrays störenden Stand der Technik gibt. Accordingly, small molecule compound metabolites are preferably composed of the following classes of compounds: alcohols, alkanes, alkenes, alkines, aromatic compounds, ketones, aldehydes, carboxylic acids, esters, amines, imines, amides, cyanides, amino acids, peptides, thiols, thioesters, phosphate esters, sulfate esters, thioethers, sulfoxides, ethers, or combinations or derivatives of the aforementioned compounds. The small molecules among the metabolites may be primary metabolites which are required for normal cell function, organ function or human or animal growth, development or health. Moreover, small molecule metabolites further comprise secondary metabolites having essential ecological function, e.g. metabolites which allow an organism to adapt to its environment. Furthermore, metabolites are not limited to said primary and secondary metabolites and further encompass artificial small molecule compounds. Said artificial small molecule compounds are derived from exogenously provided small molecules which are administered or taken up by an organism but are not primary or secondary metabolites as defined above. For instance, artificial small molecule compounds may be metabolic products obtained from drugs by metabolic pathways of the animal. In one embodiment, metabolites further include peptides, oligopeptides, polypeptides, oligonucleotides and polynucleotides, such as RNA or DNA. More preferably, a metabolite has a molecular weight of 50 Da (Dalton) to 30,000 Da, most preferably less than 30,000 Da, less than 20,000 Da, less than 15,000 Da, less than 10,000 Da, less than 8,000 Da, less than 7,000 Da, less than 6,000 Da, less than 5,000 Da, less than 4,000 Da, less than 3,000 Da, less than 2,000 Da, less than 1,000 Da, less than 500 Da, less than 300 Da, less than 200 Da, less than 100 Da. Preferably, a metabolite has, however, a molecular weight of at least 50 Da. Most preferably, a metabolite in accordance with the present invention has a molecular weight of 50 Da up to 1,500 Da.

Preferably metabolites can be grouped and analyzed according to their chemical structure or their biological function in acylcarnitines, alcohols, aldehydes, alkaloids, amides, amines, proteinogenic amino acids, non-proteinogenic amino acids, bases, carbohydrate acids, carbohydrate alcohols, carbohydrate phosphates, carbohydrates, carboxylic acids, carnitines, carotenoids, catecholamines, catechols, ceramides, cerebrosides, chlorophylls, coenzymes, enzymes, esters, ethers, fatty acids, fatty acyls, flavonoids, gangliosides, glucosinolates, glycerolipids, glycerophospholipids, heterocycles, hormones, hydrocarbons, indoles, inorganic acids, ketones, lactones, lipids, mono- and oligosaccharides, nucleosides, nucleotides, organic acids, peptides, phenols, phosphates, phospholipids, porphyrins, prenol lipids, prostaglandins, proteins, saccharolipids, sphingolipids, sphingomyelins, sphingosines, steroids, sterol lipids, sulfonic acids, terpenoids, tocopherols, tocotrienols and/or, vitamins. In a preferred embodiment of the invention the metabolites referred to in accordance with the present invention are small molecule compounds belonging to one or more of the above cited metabolite groups. In a further preferred embodiment the metabolites referred to in accordance with the present invention are small molecule comprising the groups of fatty acids and/or amino acids and/or organic acids.

The metabolite may be determined by its specific physical or chemical properties. Such properties include, e.g., molecular weight, viscosity, density, electrical charge, spin, optical activity, colour, fluorescence, chemoluminescence, elementary composition, chemical structure, capability to react with other compounds, capability to elicit a response in a biological read out system (e.g., induction of a reporter gene) and the like. Accordingly, the metabolic data to be determined by the method of the present invention are values for those parameters or values related thereto. The values shall indicate at least one identifier for a metabolite. In case of the molecular weight, such an identifier may be the mass-to-charge ratio (m/z) as determined by a mass spectrometry device. A further identifier of a metabolite may be its chromatographic retention time if chromatography coupled mass spectrometry is used for the generation of the metabolic data. Further, the value shall indicate a signal intensity observed for the identifier.

Devices which are suitable for determining metabolic data are, preferably, nuclear magnetic resonance (NMR), magnetic resonance imaging (MRI), Fourier transform infrared analysis (FT-IR), ultra violet (UV) spectroscopy, refraction index (RI), fluorescent detection, radiochemical detection, electrochemical detection, light scattering (LS), dispersive Raman spectroscopy or flame ionisation detection (FID). Most preferably, the device is a mass spectrometry (MS) device optionally coupled to a liquid (LC) or gas chromatography device (GC). Other mass spectrometry devices are direct infusion mass spectrometry or Fourier transform ion-cyclotrone-resonance mass spectrometry (FT-ICR-MS), capillary electrophoresis mass spectrometry (CE-MS), high-performance liquid chromatography coupled mass spectrometry (HPLC-MS), quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time of flight mass spectrometry (TOF). Said techniques are disclosed in, e.g., Niessen, Journal of Chromatography A, 703, 1995: 37-57, US 4,540,884 or US 5,397,894, the disclosure content of which is hereby incorporated by reference. These techniques are well known to the person skilled in the art and can be applied without further ado. The devices used for determining the metabolic data generate a digital signal (instrument file), from which the metabolite data can be extracted.

The devices used for determining the metabolic data are, preferably, small scale instruments being capable of determining metabolic data of only a subset of metabolites, i.e. the relevant metabolites for the diagnosis. Accordingly, the means for determining metabolic data, preferably, determine only metabolic data of the relevant metabolites and are, thus, less time and cost intensive than the conventional large scale devices usually used for diagnostic approaches. Small scale devices may be micro-arrays or miniaturised chromatographic or electrophoresis devices, such as devices for miniaturised capillary electro-chromatography. More preferably, the small scale device is portable and can be used in near-patient approaches.

The term "sample" as used herein refers to a sample of a tissue, an organ or a body fluid of said subject or, in case of plants, a sample of a plant tissue, seed, or other part of the plant. The sample according to the present invention, preferably, includes sudor, skin, breath or other indicative organ or body samples.

The term "subject" refers to biological organisms, preferably to animals or plants. Preferably, the animal is a mammal and, most preferably, a human.

The metabolic data obtained from the aforementioned means are subsequently converted in an input format. A suitable input format, preferably, comprises metabolic data which have been depleted of noising, inconsistent or otherwise inferior data. The input format aggregates the single metabolite information from preferably one device and two or more extraction steps.

A series of rules can be defined to reduce the high dimensional space of the digital signal of the device, and to assess the identity and quantity of each individual metabolite, e.g., baseline removal, de-noising and smoothing electrical and chemical noise, retention time alignment and mass/charge peak alignment. A subsequent step to the metabolite detection consists of normalization and quantification. Meticulous quality control, cleaning and transformation of these processing steps prior to data analysis is essential to distinguish the correct signals from background noise and a prerequisite for an accurate metabolite identification and quantification. The combination and/or sum of all metabolites represent the input format.

Moreover, the metabolic data should be normalized in order to allow a conclusive comparison, in particular, for quantitative evaluation. The data validation and data normalization can be carried out by various algorithms depending on the kind of data to be investigated. Validation as used herein means that the either the entire raw data or a subset thereof, preferably, the raw data of the informative metabolites, are investigated for inconsistencies. The investigation is, preferably, carried out by a computer implemented algorithm. The algorithm, preferably, applies rules from, e.g., a rule database to the raw data. Said rules are capable of identifying and/or subsequently confirming or invalidating raw data. The rules, preferably, define limits for the expected and/or derived values of the raw data based on known reference data. Such reference data may, e.g., be derived from standards (compounds) which are indicative for proper operation of the devices and the entire process of data determination. Further, reference data may be derived from standards (compounds) which are used to calculate the values of the raw data of the metabolites, e.g., by extrapolation techniques. The type of standards may depend on the method applied to determine the metabolic data of the metabolites. If, for instance, chromatography coupled mass spectrometry, such as LCMS or GCMS, is used, the standards may be retention time standards. Moreover, in addition or instead the selection of specific relevant (informative) metabolites yielding specific relevant metabolic data, the said data belonging to the relevant metabolites for the diagnosis may also be identified and/or marked during conversion of the data into the input format. More preferably, the input format shall only contain the informative metabolic data. The algorithms for conversion are implemented into the system by the "means for converting the metabolic data into an input format" referred to in accordance with the present invention. Thus, the said means, preferably, comprise a database comprising rules for data validation and/or normalization and computer program code implemented on a computer for carrying out the aforementioned algorithm on the metabolic data.

The system further comprises "at least one database comprising reference metabolic data in a format which allows comparison to the input format". Said term relates to one or a plurality of databases comprising reference metabolic data. Reference metabolic data for determining a physiological status are those which allow allocating a certain physiological status to the subject to be investigated by the system of the present invention. Preferably, if a disease or disorder is to be diagnosed by the system of the present invention, the reference metabolic data are metabolic data which specifically indicate the disease or disorder in the subject. Similarly if a predisposition or a risk for a disease or disorder is to be diagnosed by the system of the present invention, the reference metabolic data are metabolic data which specifically indicated the risk or the prevalence for a disease or disorder in the subject. Alternatively, the metabolic data may characterise a healthy subject with respect to the said disease or disorder. Therefore, the reference metabolic data are, preferably, either data obtained from a subject suffering from the disease to be diagnosed or from a subject known not to suffer from said disease. Reference metabolic data can be uploaded or integrated from different sources like literature, clinical trials and other sources, which included valid relations between metabolic data and disease or health status. The reference data shall be in a format which allows comparison with the metabolic data to be investigated. Accordingly, the reference metabolic data are, preferably, in the input format, too. However, the reference metabolic data may also be stored in the database in an alternative format provided that the format allows for comparison with data in the aforementioned input format.
Moreover, the system, preferably, encompasses further data, relating to additional subject symptoms, life style, age, gender, height, weight, health status, medicinal history (i.e. present and past illness, family diseases or medication). Biological expert knowledge, preferably, on biochemical pathways and/or metabolism, can be used in addition to fill the database with pertinent information on single diseases and/or disease markers increasing the accuracy and precision of a diagnostic result. The aforementioned data are, preferably, stored in a separate database being operatively linked to the system. The data stored in the said further database will be allocated to the results obtained by the comparison and provided in a suitable output format. Moreover, the result of the comparison may be further interpreted by an expert algorithm based on the aforementioned data. The expert algorithm will then provide the results of the said interpretation. For example, it is envisaged that the result of the metabolic data comparison (e.g., the presence of a certain disease status) is confirmed by the additional data (e.g., by general data on biochemical pathways or apparent symptoms of the subject). Alternatively or in addition, it is envisaged that the system will generate an output format comprising the results of the comparison of metabolic data and recommendations for, e.g., life style based on the said additional data (e.g., if the metabolic data comparison is indicative for diabetes, the output format shall also recommend a suitable diet). The additional data may, alternatively, also be stored in the at least one database comprising the reference metabolic data. In one aspect the provision of the described database comprising reference metabolic data and optionally further data as described, alone or preferably in combination with the other means of the system for determining a physiological status of a subject provide a new business method for the diagnostic sector.

The term "means for comparing the metabolic data in the input format to the reference metabolic data" relates, preferably, to a computer implemented algorithm which allows identifying differences or similarities between the metabolic data and the reference metabolic data. In principle, any statistical test which allows determining whether metabolic data will vary significantly between different data sets is suitable for carrying out the aforementioned comparison. More preferably, suitable techniques include a pattern recognition algorithm and/or classification algorithm and/or decision rules and/or a statistical test algorithm and/or a multivariate algorithm, e.g., Principal Component Analysis (PCA), Simple Component Analysis (SCA), Independent Component Analysis (ICA), Principal Component Regression (PCR), Partial Least Squares (PLS), PLS Discriminant Analysis (PLS-DA), Support Vector Machines (SVM), partical Swarm Optimization (PSO), Neural Networks, Bayesian Networks, Bayesian Learning Networks, Mutual Information, Backpropagation Networks, symmetrical Feed-Forward Networks, Self-Organizing Maps (SOMs), Genetic Algorithms, Hierarchical or K-Mean Clustering, Anova, Student's t-Test, Kruskal-Wallis Test, Mann-Whitney Test, Tukey-Kramer Test or Hsu's Best Test. Preferably, the comparison of data as described above can be applied to determine differences or similarities between metabolic data reflecting differences in the qualitative, semiquantitative or quantitative composition of the metabolites comprised by the samples which have been investigated, i.e. the test and reference samples. Thus, the means for comparison, preferably, comprise a computer for carrying out the comparison and shall have access to the at least one database comprising reference metabolic data in a format which allows comparison to the input format. It is to be understood that the computer implemented algorithm for comparison does not necessarily have to be implemented physically into the same device as the database. It is to be understood that the means for comparing may receive queries from a plurality of different means for determination of metabolic data. Preferably the system shall be an open system which can constantly add new information about relations between metabolites and a physiological status allowing an up to date interpretation of metabolic data. For example data from clinical studies, which link certain metabolic changes to certain disease states may be added to extract as much as possible valuable information for the metabolic data.
The term "output format" as used herein refers to a format, e.g., a data table, which indicates the differences or similarities in the metabolic data. Moreover, the output format may indicate the metabolites which are changed and the degree of the said change by reciting either relative or absolute amounts optionally with respect to the corresponding reference values. Moreover, the output format may comprise further information on the disease to be diagnosed as well as therapeutic suggestions obtained from a database containing said information. The type of output format will depend on the user who receives it. For example, a medical practitioner may require information on the changes in the metabolites, whereas a patient using the system for a self-diagnosis or monitoring may require further information on the disease and/or possible therapies.

Thus, the "means for converting the results of the comparison in a suitable output format" shall be capable of converting the resulting data of the comparison into a format as described above. Preferably, the said means are also capable of allocating information stored in a further database, e.g., disease related information or therapeutic information, to the said results. The algorithms used for carrying out the conversion of the results of the comparison and the allocation of the information are, preferably, computer implemented into the aforementioned means. Moreover, the aforementioned means may be operatively linked to an expert system.

Advantageously, the system of the present invention allows for a fast and nevertheless reliable analysis of metabolic data. The analysis can be applied for diagnosing diseases as described above. However, as disclosed elsewhere in this specification, the system may, in principle, be used for other applications as well. The system avoids the drawbacks of the prior art technologies by optimizing the functional resources. It combines the reliability and broadness of large scale systems and, thus, allows even analysis of more complex metabolic patterns comprising several metabolic changes with the velocity of near-patient systems providing the diagnostic results quickly. Moreover, the system is cost-efficient, since the expensive means for comparison and the database are, in principle, required only once while serving to process the queries of a plurality of different users. At the users end, only means for determining the metabolic data and, optionally, a computer terminal for receiving the results in the output format are required. Due to miniaturization and restriction to the determination of metabolic data from the informative metabolite, only, the said means can be provided on large scale and rather inexpensive.

For example, a system of the present invention comprises a portable detection device for metabolic data (Figure.; (101)), such as a miniaturized mass spectrometry or a device for miniaturized capillary electro-chromatography. The metabolic data obtained from a sample by the said device will be converted into a suitable input format and transmitted, e.g., via the internet, to a device for analysing the data (Figure; (103), e.g., a computer. Alternatively, the metabolic data may be transmitted to the analysis device (Figure; (103)) and converted into an input format by the said device. The analysis device (Figure; (103)) will carry out the comparison of the metabolic data with the referenced data stored in an accompanying database (Figure; (104)). The database may be included in the analysis device, e.g., a computer, or may be located at a different location. The computer may also retrieve further information relating to the sample or the subject from which the sample was obtained, e.g., medicinal history data or life style data from the same or a different database. The computer will based on these data generate a suitable output format. This output format will be provided to the user, e.g., to the subject to be analyzed or to a medical practitioner investigating the said subject. The output format shall comprise at least information (i) as to whether the metabolic data obtained from the sample match with metabolic reference data and (ii) information on the physiological status which is indicated by the said reference data. Further information may be recommendations with respect to the physiological status, such as life style recommendations for the subject.

In the following, particularly preferred embodiments of the diagnostic system of the present invention are described:
In a preferred embodiment of the system, said means for determining metabolic data comprise means for automated sample preparation and analysis. More preferably, the automated sample preparation and analysis comprises solid-phase extraction, chromatographic separation or mass spectrometric detection or combinations thereof. Automated preparation and/or analysis can be implemented by means of robotics and by computer-based control systems.
In another preferred embodiment of the system, the metabolic data are quantitative metabolic data. The term "quantitative" as used herein includes absolute or relative quantitative data as well as semi-quantitative data. The quantification of the metabolic data is, more preferably, performed with isotopically labelled internal standards. Such a procedure will yield quantitative data being normalized with respect to an internal standard and, thus, relative quantitative data. For the said standard, isotopes are to be used as label which shall not occur in the metabolites to be analyzed, e.g., isotopes which do not occur naturally in biological samples. Semi-quantitative data as referred to herein shall merely indicate a change in the amount of the metabolite from which the data are obtained, i.e. an increase, decrease or steady state of the amount of the said metabolite.
In a preferred embodiment of the system, the means for converting the metabolic data comprise means for an automatic validation of the data. More preferably, the validation is performed with an unsupervised validation algorithm.
In a preferred embodiment of the system, the means for converting the metabolic data comprise means for automated feature extraction from multidimensional chromatographic and/or mass spectrometric raw data. Feature extraction refers to any method to identify the most useful and best describing variables for each metabolite out of the raw data, and to integrate the sum of single electrical signals into one value for each metabolite.
In a further preferred embodiment of the system, said system is automatically checked before analysis by comparing the operation parameters of the system with defined instrument reference values. The defined instrument reference values, preferably, indicate proper operation of all components of the system. If the operation parameters do not correspond to the reference parameters (e.g., are not identical or without a defined limits), the operation of the entire system will be aborted or data will be invalidatedand/or cancelled.
In a preferred embodiment of the system, at least the means of (a) are physically separated from the means of (b), (d), and (e) and the database of (c). For a portable system as referred to above, the means for determining the metabolic data are necessarily in close proximity to the sample and, preferably, even to the subject from which the sample shall be obtained. Thereby, it can be prevented that the metabolic composition of the sample which shall reflect the metabolic composition in the subject whose physiological status is to be investigated will be altered due to degradation or chemical modification of the metabolites. It is to be understood that an altered composition of the sample would also result in altered metabolic data which are no longer representative for the physiological status of the subject. Alternatively, an alteration of the metabolite composition of the sample can be prevented by additives such as stabilizers and by a careful handling of the sample. More preferably, the other components of the system may be physically separated from each other as well.

In light with the foregoing, it is to be understood that in another preferred embodiment of the system, the means of (a) and at least the means of (b) are operatively linked via the internet. Thereby, the means for determining the metabolic data, e.g., a portable detection device for the metabolic data, can be operatively linked without a wire based connection.

The present invention also relates to a method for determining a physiological status in a subject comprising:
(a) determining metabolic data from a sample of a subject whose physiological status is to be determined;
(b) converting the metabolic data into an input format;
(c) comparing the metabolic data of (b) with reference metabolic data which are indicative for the physiological status which are stored in at least one database;
(d) converting the results of the comparison of (c) into an output format.

The above explanations and preferred embodiments implemented by the system of the present invention are also, preferably, implemented in the method of the present invention.

Thanks to the method of the present invention, a reliable and nevertheless time and cost efficient determination of a plurality of different physiological states based on metabolic data has become possible. Specifically, the methods allows for a reliable, metabolome based diagnosis of subjects which may not have immediate access to hospitals or medical facilities. Due to the method of the present invention, resident medical practitioners will have readily access to the reliable and precise analysis tools including the comprehensive metabolomic databases of large medical facilities. Moreover, even subjects which are physically separated from an access to such facilities, e.g., being in the outback, can be investigated, e.g., via internet access. It is to be understood that the aforementioned advantages apply for the other methods referred to below accordingly. Specifically the central position of the database allows for the systematic and expert guided addition of relevant information supporting the interpretation of metabolic information from the subject.

Further approaches, in which the systems of the present invention may be used, are as follows. All definitions and explanations made for the above systems and methods apply mutatis mutandis.

The present invention also relates to a system for assessing toxicity of a compound comprising
(a) means for determining metabolic data from a sample of a subject which has been contacted to a compound suspected to be toxic;
(b) means for converting the metabolic data into an input format;
(c) at least one database comprising reference metabolic data in a format which allow comparison to the input format;
(d) means for comparing the metabolic data in the input format to the reference metabolic data comprised by the at least one database; and
(e) means for converting the results of a comparison carried out by the means of (d) into an output format.

The term "assessing toxicity" as used herein refers to assessing whether a compound is capable of eliciting a toxic reaction in the subject. The term "accessing toxicity" further comprises the alignment or classification of the toxic reaction in relation to known or predefined toxicity reactions. Toxic reactions as referred to in accordance with the present invention are biological reactions which affect a cell, a tissue or an organ of a subject in a harmful manner by interfering with their normal physiological functions. In severe forms, toxicity will result in organ failure or even mortality. Toxicity may be indicated by IC₅₀ values well known in the art. As will be understood readily by those skilled in the art, a toxicity assessment is usually not intended to be correct for 100% of the treated subjects. The term, however, requires that the compound will be toxic for at least a statistically significant portion of subjects. Whether a portion is statistically significant can be determined without further ado by the person skilled in the art using various well known statistic evaluation tools, preferably, those mentioned above.

The present invention also encompasses a method for assessing toxicity of a compound comprising
(a) determining metabolic data from a sample of a subject which has been contacted to a compound suspected to be toxic;
(b) converting the metabolic data into an input format;
(c) comparing the metabolic data of (b) with reference metabolic data which are indicative for toxicity and which are stored in at least one database;
(d) converting the results of the comparison of (c) into an output format.

The present invention, thus, relates to a system for determining a mode of action of a compound comprising
(a) means for determining metabolic data from a sample of a subject which has been contacted to a compound;
(b) means for converting the metabolic data into an input format;
(c) at least one database comprising reference metabolic data in a format which allows comparison to the input format;
(d) means for comparing the metabolic data in the input format to the reference metabolic data comprised by the at least one database; and
(e) means for converting the results of a comparison carried out by the means of (d) into an output format.

The term "determining a mode of action" refers to identifying how a compound affects an organism on the molecular level. A mode of action is characterized by the biochemical reactions which occur in an organism. The biochemical reactions, also referred to as biochemical pathways, may be part of the metabolism of an organism or may serve for signal transduction. The biochemical reactions and particularly those of the metabolism are to a large extent well understood and known in the art. However, even if a metabolic pathway and its adducts, intermediates, and products are not characterized in detail, a change in the metabolic reactions will become apparent in the metabolic data. For example, the metabolic data obtained from a sample of an organism which has been contacted with a compound known to affect a metabolic pathway, will reflect the altered metabolism by changes in the metabolite composition and the amounts of the metabolites also referred to as the metabolite profile. Thus, a compound known to affect a metabolic pathway shall produce a specific metabolite profile. The same applies for a compound affecting a signal transduction pathway. The metabolite profile or parts thereof can serve as a marker for a mode of action of a compound. Accordingly, if a compound with an unknown mode of action is investigated by the system of the present invention, its mode of action can be determined by way of comparison to metabolic profiles of compounds having a known mode of action stored in the database. Determining the mode of action of a compound may, in case of pharmaceutical, herbicidal, pesticidal, or insecticidal compositions help to prioritise compounds or to improve their properties and to avoid undesired side effects.

Finally, the present invention relates to a method for determining a mode of action comprising
(a) determining metabolic data from a sample of a subject which has been contacted to a compound;
(b) converting the metabolic data into an input format;
(c) comparing the metabolic data of (b) with reference metabolic data which are indicative for a mode of action and which are stored in at least one database;
(d) converting the results of the comparison of (c) into an output format.

All references referred to above are herewith incorporated by reference with respect to their entire disclosure content as well as their specific disclosure content explicitly referred to in the above specification.

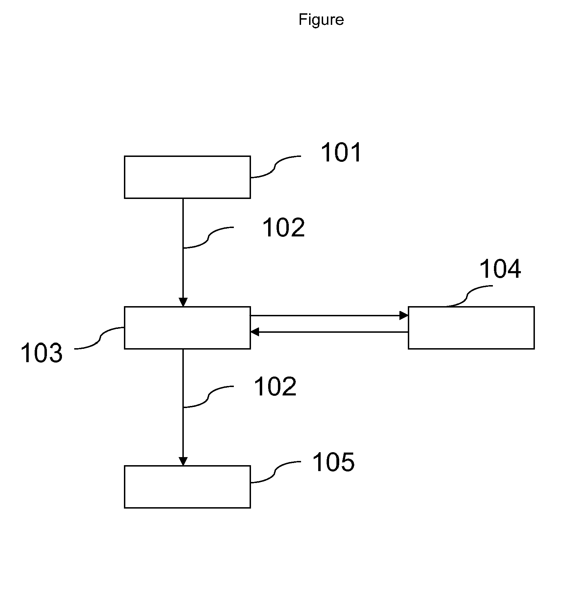

The figure shows a schematic view of the system of the present invention. A device for compound detection (101), e.g., a mass spectrometry device, is used to obtain metabolic data from the informative metabolites comprised by a test sample of a subject. The metabolic data are converted into an input format and submitted via, e.g., the internet (102) to a device located at a distance used for the analysis of the data (103), i.e. for the comparison of the metabolic data to the reference metabolic data stored in a database (104) by using a computer implemented algorithm. The results of the comparison are provided in a suitable output format and submitted (102) to a terminal (105) where the output format is displayed upon provision of the access key by the user.

## Claims

1. System for determining a physiological status of a subject comprising operatively linked to each other:
(a) means for determining metabolic data from a sample of a subject whose physiological status is to be determined;
(b) means for converting the metabolic data into an input format;
(c) at least one database comprising reference metabolic data in a format which allows comparison to the input format;
(d) means for comparing the metabolic data in the input format to the reference metabolic data comprised by the at least one database; and
(e) means for converting the results of a comparison carried out by the means of (d) into an output format.

2. The system of claim 1, wherein said means for determining metabolic data comprise means for automated sample preparation and analysis.

3. The system of claim 2, wherein the automated sample preparation and analysis comprises solid-phase extraction, chromatographic separation or mass spectrometric detection or combinations thereof.

4. The system of any one of claims 1 to 3, wherein the metabolic data are quantitative metabolic data.

5. The system of claim 4, wherein the quantification is performed with isotopically labelled internal standards.

6. The system of any one of claims 1 to 5, wherein the means for converting the metabolic data comprise means for an automatic validation of the data.

7. The system of claim 7, wherein the validation is performed with an unsupervised validation algorithm.

8. The system of any one of claims 1 to 7, wherein the means for converting the metabolic data comprise means for automated feature extraction from multidimensional chromatographic and/or mass spectrometric raw data.

9. The system of any one claims 1 to 8, wherein the system is automatically checked before analysis by comparing the status parameters of the system with defined instrument reference values.

10. The system of any one of claims 1 to 9, wherein at least the means of (a) are physically separated from the means of (b), (d), (e) and the database of (c).

11. The system of any one of claims 1 to 10, wherein the means of (a) and at least the means of (b) are operatively linked via the internet.

12. The system of any one of claims 1 to 11, wherein said reference metabolic data are indicative for the physiological status to be diagnosed.

13. The system of any one of claims 1 to 12, wherein the physiological status is a disease status.

14. The system of any one of claims 1 to 13, wherein the metabolic data are compared to the reference database by means of pattern recognition algorithm and/or classification algorithm and/or decision rules.

15. A method for determining the physiological status of a subject comprising:
(a) determining metabolic data from a sample of a subject whose physiological status is to be determined;
(b) converting the metabolic data into an input format;
(c) comparing the metabolic data of (b) with reference metabolic data which are indicative for a physiological status and which are stored in at least one database;
(d) converting the results of the comparison of (c) into an output format.

16. The method of claim 15, wherein said method is assisted by automation.

17. The method of claim 15 or 16, wherein said determining metabolic data comprise sample preparation and analysis.

18. The method of claim 17, wherein the sample preparation and analysis comprises solid-phase extraction, chromatographic separation or mass spectrometric detection or combinations thereof.

19. The method of any one of claims 15 to 18, wherein the metabolic data are quantitative metabolic data.

20. The method of claim 19, wherein the quantification is performed with isotopically labelled internal standards.

21. The method of any one of claim 15 to 20, wherein said converting the metabolic data comprises an automatic validation of the data.

22. The method of claim 21, wherein the validation is performed with an unsupervised validation algorithm.

23. The method of any one of claim 15 to 22, wherein said converting the metabolic data comprises automated feature extraction from multidimensional chromatographic and/or mass spectrometric raw data.

24. The method of any one of claims 15 to 23, wherein said reference metabolic data are indicative for the physiological status to be diagnosed.

25. The method of any one of claims 15 to 24, wherein the physiological status is a disease status.

26. The method of any one of claims 15 to 25, wherein the metabolic data are compared to the reference database by means of pattern recognition algorithm and/or classification algorithm and/or decision rules.
